Europäisches Patentamt

European Patent Office

Office européen des brevets

(11) Numéro de publication : **0 399 920 B1**

## (12) FASCICULE DE BREVET EUROPEEN

(45) Date de publication du fascicule du brevet :
**28.10.92 Bulletin 92/44**

(51) Int. Cl.⁵ : **A61F 2/36**

(21) Numéro de dépôt : **90420218.1**

(22) Date de dépôt : **09.05.90**

(54) **Tige intramédullaire pour prothèse vissée.**

(30) Priorité : **23.05.89 FR 8906975**

(43) Date de publication de la demande :
**28.11.90 Bulletin 90/48**

(45) Mention de la délivrance du brevet :
**28.10.92 Bulletin 92/44**

(84) Etats contractants désignés :
**AT BE CH DE DK ES FR GB GR IT LI LU NL SE**

(56) Documents cités :
**EP-A- 0 038 897**
**EP-A- 0 222 979**
**DE-U- 8 611 697**
**FR-A- 2 628 628**

(73) Titulaire : **Bousquet, Gilles**
**Chemin de Marandon**
**F-42100 Saint-Etienne (FR)**
Titulaire : **Rambert, André**
**Les Fontanelles 10 bis, rue du Docteur**
**Bonhomme**
**F-69003 Lyon (FR)**

(72) Inventeur : **Bousquet, Gilles**
**Chemin de Marandon**
**F-42100 Saint-Etienne (FR)**
Inventeur : **Rambert, André**
**Les Fontanelles 10 bis, rue du Docteur**
**Bonhomme**
**F-69003 Lyon (FR)**

(74) Mandataire : **Maureau, Pierre et al**
**Cabinet GERMAIN & MAUREAU B.P. 3011**
**F-69392 Lyon Cédex 03 (FR)**

EP 0 399 920 B1

Jouve, 18, rue Saint-Denis, 75001 PARIS

## Description

La présente invention concerne une tige intramédullaire pour prothèse vissée.

Par le brevet français 80 10134, on connaît une tige intramédullaire à filetage tronconique destinée à être vissée dans le canal médullaire du fémur réséqué dont la tête et le col doivent être remplacés par cette prothèse.

Le vissage de sa tige dans le canal médullaire du fémur procure à cette prothèse une grande stabilité qu'il est encore possible d'améliorer en recouvrant ses filets d'une mince couche d'alumine dont la présence favorise l'ostéogène.

Cependant, pour réussir la pose d'une prothèse de ce type, il est indispensable de la bloquer, en fin de vissage, avec un couple assez important, de l'ordre de 60 à 70 N.m. Il est donc indispensable de prendre certaines précautions lors de leur pose, surtout lorsqu'un praticien les utilise pour la première fois. En outre, la bonne intégration de cette tige à l'os impose, en cas d'obligation de retrait, un couple de déblocage supérieur au couple de blocage et beaucoup de clients potentiels sont effrayés par la perspective du dévissage d'une prothèse placée depuis plusieurs années, tant il est vrai qu'un chirurgien pourra toujours être confronté à une telle réalité pour diverses raisons, indépendantes d'un mauvais comportement de la prothèse.

La présente invention vise à pallier cet inconvénient. A cet effet, dans la tige intramédullaire qu'elle concerne et qui est du type précité, il est prévu, d'une part, un alésage axial débouchant au moins à son extrémité proximale ou externe dans la paroi de révolution duquel est découpée au moins une languette liée à cette paroi par son extrémité la plus interne et dont la face interne présente une rampe de pente orientée vers l'extérieur, en direction de son extrémité libre et, d'autre part, un organe d'impactage déplaçable axialement depuis l'extrémité extérieure de l'alésage précité et dont chaque secteur ayant la même position angulaire qu'une languette présente une rampe de pente correspondant à celle de la languette associée, de manière à permettre, par son déplacement axial en direction de l'extrémité distale de la tige, un déplacement radial vers l'extérieur de l'extrémité libre de chaque languette, augmentant ainsi le couple de dévissage de cette tige.

Ainsi, il n'est pas nécessaire de bloquer cette tige en exerçant un couple important qui risquerait de provoquer l'éclatement de l'os dans lequel elle est vissée, ce blocage pouvant être obtenu par simple déplacement axial de l'organe d'impactage. Inversement, si elle doit être dévissée, un simple déplacement axial en sens inverse de l'organe d'impactage est suffisant pour abaisser considérablement son couple de dévissage.

Généralement, les tiges vissables présentent, à leur extrémité extérieure ou proximale, un bossage tronconique destiné à recevoir l'embase de l'élément céphalique qui leur est associé.

Suivant une première forme d'exécution de l'invention, le bossage tronconique destiné à recevoir l'élément céphalique est solidaire de l'extrémité proximale de la tige intramédullaire filetée et il est prévu, en dessous de ce bossage, six fentes longitudinales de répartition angulaire régulière délimitant six secteurs de 60° dont trois, non adjacents et destinés à constituer des languettes, ont leur extrémité la plus proche du bossage libérée par une fente en arc de cercle, l'organe d'impactage étant constitué par une pièce cylindrique tubulaire présentant, sur sa face externe de révolution, trois secteurs de troncs de cône disposés à 120° l'un de l'autre et dont chacun est appliqué contre l'une des languettes précitées lors de son engagement dans l'alésage axial de la tige, l'alésage de cet organe d'impactage étant taraudé pour permettre son déplacement axial en sens inverse en cas de dévissage nécessaire de la tige.

Après vissage de cette tige avec un couple léger de l'ordre de 20 N.m, il est facile d'enfoncer l'organe d'impactage sur la course désirée, avec un outil muni d'un épaulement ou d'une collerette apte à prendre appui sur le bossage tronconique de la tige en fin de course de l'organe d'impactage.

De préférence, pour garantir l'immobilisation axiale de l'organe d'impactage, l'extrémité débouchante ou proximale de l'alésage de la tige est taraudée et il est prévu une vis tubulaire de blocage apte à être vissée dans cet alésage jusqu'à son contact avec l'organe d'impactage, l'alésage de cette vis étant taraudé pour permettre la mise en place de la vis de blocage de l'embase de l'élément céphalique.

Suivant une variante d'exécution de cette tige intramédullaire, d'une part, l'extrémité distale ou non débouchante de son alésage est taraudée et son extrémité débouchante ou proximale est tronconique et, dans la paroi de cette extrémité, sont découpées, par six fentes longitudinales débouchantes de répartition angulaire régulière, six languettes et, d'autre part, l'organe d'impactage est constitué par un bossage inférieur tronconique de pente correspondant à celle de l'extrémité proximale de l'alésage de la tige, solidaire d'une pièce tubulaire de liaison qui porte, coaxialement à lui, un bossage tronconique supérieur destiné à recevoir l'élément céphalique de la prothèse, l'alésage de cette pièce étant destiné au passage d'une vis de fixation de cet élément céphalique, apte à être vissée dans l'extrémité distale taraudée de l'alésage axial de la tige, tandis que les deux bossages, inférieur et supérieur de cette pièce tubulaire de liaison, sont séparés l'un de l'autre par une collerette destinée à limiter l'enfoncement du bossage inférieur dans l'extrémité tronconique de l'alésage de la tige à la valeur correspondant a l'expansion radiale désirée des languettes.

De préférence, pour permettre son retrait et, par conséquent celui de la tige intramédullaire, au moins l'extrémité supérieure de l'alésage de la pièce tubulaire de liaison est taraudée.

Suivant une autre variante d'exécution de cette tige intramédullaire, d'une part, l'organe d'impactage déplaçable axialement est constitué par l'extrémité libre tronconique d'un prolongement cylindrique lisse d'une vis destinée à être vissée dans l'extrémité proximale taraudée de son alésage axial et, d'autre part, chaque languette porte, sur sa face interne, une saillie radiale destinée à être engagée par l'extrémité tronconique du prolongement lisse de la vis, en fin de vissage.

De préférence, l'extrémité proximale de la vis dont un prolongement d'extrémité tronconique constitue l'organe d'impactage présente un alésage axial taraudé destiné à recevoir la vis de blocage d'une tête céphalique.

De toute façon, l'invention sera bien comprise à l'aide de la description qui suit, en référence au dessin schématique annexé représentant, à titre d'exemples non limitatifs, deux formes d'exécution de cette tige intramédullaire pour prothèse vissée, dans le cas de son application à l'élément fémoral d'une prothèse de hanche :

Figures 1 à 3 sont des vues en coupe axiale d'une première forme d'exécution de cette tige intramédullaire illustrant le mode d'exécution de son impactage après vissage ;

Figure 4 est une vue similaire aux figures 1 à 3 montrant la tige intramédullaire des figures 1 à 3, après montage des pièces destinées à compléter cette tige pour la constitution d'un élément fémoral de prothèse de hanche ;

Figures 5 et 6 sont des vues en coupe suivant V-V et VI-VI de figure 4 ;

Figure 7 est une vue similaire à figure 4 illustrant une variante d'exécution de la tige fémorale selon l'invention ;

Figure 8 est une vue en coupe suivant VIII-VIII de figure 7 ;

Figure 9 est une vue en coupe axiale, similaire aux figures 4 et 7, montrant une autre variante d'exécution de cette tige ;

Figure 10 est une vue en coupe transversale suivant X-X de la figure 9.

La tige intramédullaire 2 représentée sur le dessin étant destinée à la constitution d'une prothèse de hanche, cette tige porte, à son extrémité proximale, c'est-à-dire celle non engagée dans le canal médullaire du fémur auquel elle est destinée, un bossage tronconique 3 destiné à recevoir les pièces complémentaires de cet élément fémoral et comportant essentiellement une tête sphérique 4 destinée à remplacer la tête du fémur réséqué, un col 5 destiné également à remplacer le col de ce fémur et une embase 6 présentant un évidement tronconique 6a débouchant dans sa face inférieure et de forme complémentaire de celle du bossage 3 de la tige 2, de manière à pouvoir être engagée sur ce bossage et assurer ainsi la fixation stable du col 5 et de la tête sphérique 4 par rapport à la tige 2. Cette fixation stable est généralement garantie par la mise en place d'une vis de blocage 7 apte à être vissée dans la tige 2 ou dans un organe solidaire de cette dernière.

Dans l'exemple illustré sur le dessin, la tige intramédullaire 2, qui est du type vissé, porte un filet extérieur 2a de type tronconique, cette forme étant connue pour améliorer le contact intime de la tige avec la diaphyse de l'os et, par conséquent, l'ostéogénèse.

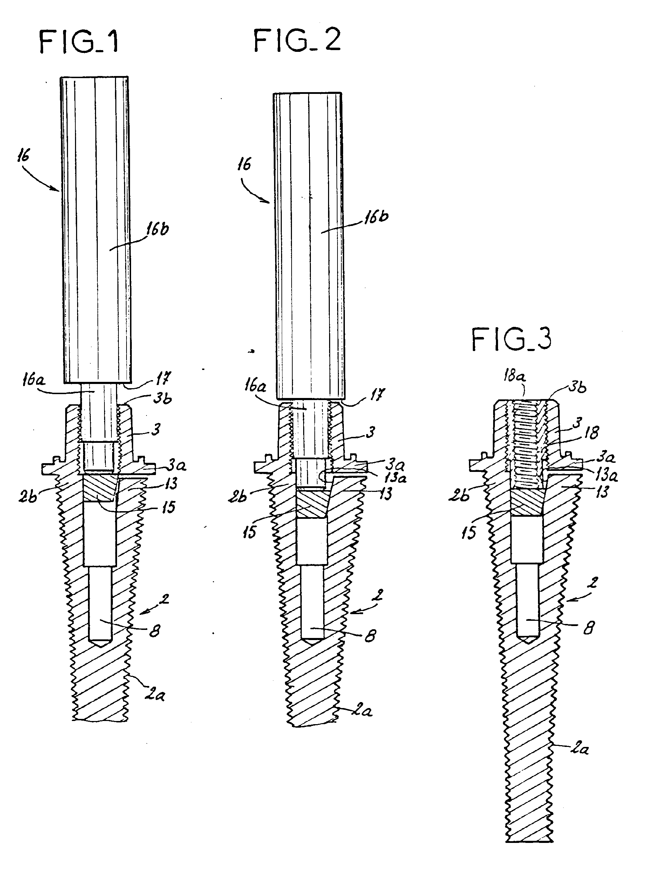

Dans la forme d'exécution illustrée sur les figures 1 à 6, le bossage tronconique 3 est solidaire de la tige 2 dont elle fait partie intégrante et constitue l'extrémité proximale 2b.

Selon l'invention, l'extrémité proximale 2b de la tige 2 présente un alésage axial 8 débouchant à cette extrémité proximale, c'est-à-dire dans la face supérieure libre du bossage 3. En outre, la paroi de la tige 2 entourant l'alésage 8 est découpée, au moins sur un tronçon supérieur de la tige 2 situé en dessous de l'embase 3a du bossage 3, en secteurs de troncs de cône égaux (six dans cet exemple), de répartition angulaire régulière, par des fentes longitudinales 9 qui, dans cet exemple, sont décalées angulairement de 60° les unes par rapport aux autres. En outre, un sur deux des secteurs de troncs de cône a son extrémité supérieure rendue libre par une fente périphérique 11 en arc de cercle. Les fentes 9 et 11 ont donc pour effet de découper, dans le tronçon précité de la paroi de l'extrémité proximale 2b de la tige 2 entourant l'alésage 8, trois secteurs de troncs de cône 12 immobiles, c'est-a-dire liés par leurs deux extrémités à la paroi de la tige 2 et trois secteurs de troncs cône 13 présentant une extrémité supérieure libre et, par conséquent, mobile radialement, notamment vers l'extérieur, c'est-à-dire dans la direction illustrée par la flèche 14. Chaque secteur 13 dont une extrémité supérieure est libre constitue une languette permettant l'expansion radiale de l'extrémité proximale 2b de la tige 2.

A cet effet, au moins au voisinage de son extrémité supérieure libre, la face interne de chaque languette 13 présente une rampe 13a inclinée de l'intérieur vers l'extérieur en direction de l'extrémité libre de la languette 13 considérée.

Enfin dans la partie supérieure de l'alésage 8 de la tige 2 est engagée une pièce d'impactage tubulaire cylindrique 15 dont la face périphérique présente trois secteurs de troncs de cône 15a dont chacun s'étend sur un arc de cercle inférieur à 60° et dont chacun est décalé angulairement de 120° par rapport à l'autre. Cet organe d'impactage 15 est engagé dans l'alésage 8 de la tige 2 de manière que chacun de ses secteurs de troncs de cône 15a soit appliqué contre l'une des languettes 13. En outre, la pente de chaque secteur de tronc de cône 15a est déterminée en fonction de la pente 13a de la languette 13 associée, de manière que, par un déplacement axial de l'organe 15 dans l'alésage 8 en direction de l'extrémité distale, la tige 2 sur une course dite course d'impactage, on provoque, par déplacement radial vers l'extérieur des extrémités libres des languettes 13a, l'expansion radiale désirée de l'extrémité proximale de la tige 2.

Comme indiqué précédemment, cette disposition permet donc, en dilatant l'extrémité proximale de la tige fémorale 2 après son vissage dans le canal médullaire du fémur dont elle est destinée à remplacer la tête et le col, d'augmenter considérablement son couple de dévissage, sans imposer, au praticien qui en effectue la pose, d'exercer un couple de vissage important et risquant éventuellement de faire éclater l'extrémité réséquée du fémur dans le canal médullaire duquel elle est engagée.

Il a été indiqué précédemment qu'avec cette disposition, il était possible de visser la tige 2 avec seulement un couple de 20N.m et d'obtenir, par le déplacement axial de l'organe d'impactage 15, un couple de dévissage de l'ordre de 60 N.m.

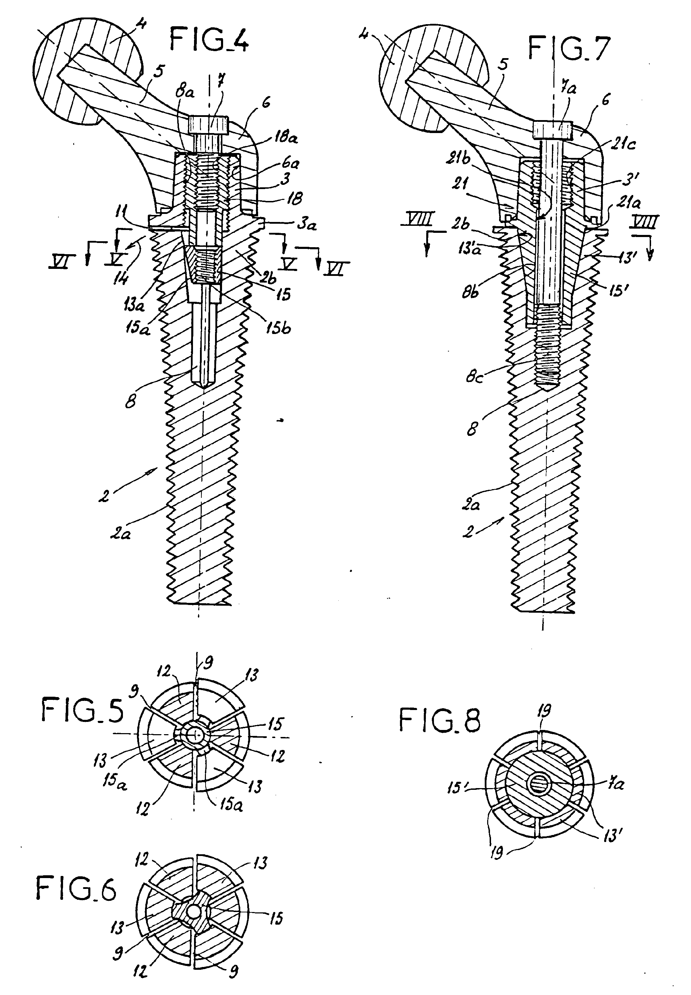

Les figures 1 à 3 illustrent un mode d'exécution de l'impactage de la tige 2 des figures 4 à 6. Comme indiqué précédemment, le couple de dévissage déterminé après impactage dépend évidemment de la course axiale que le praticien fait effectuer à l'organe d'impactage 15 après vissage de la tige 2 dans le canal médullaire du fémur réséqué. Naturellement, cette course doit être suffisante pour l'obtention du couple précité mais non excessive pour ne pas risquer, par une expansion exagérée de l'extrémité proximale 2b de la tige 2, un éclatement de l'extrémité correspondante du fémur. Dans l'exemple illustré par les figures 1 et 2, il est prévu d'utiliser un outil d'impactage 16 de forme sensiblement cylindrique qui présente une partie de faible diamètre 16a destinée à être engagée dans l'extrémité libre du canal 8 et une partie supérieure de plus grand diamètre 16b destinée à servir de poignée, ces deux parties étant séparées l'une de l'autre par un épaulement 17 apte à prendre appui sur la face supérieure du bossage 3 entourant l'extrémité débouchante de l'alésage 8. On conçoit aisément qu'en frappant sur l'extrémité libre de l'outil 16, le praticien peut provoquer l'enfoncement de l'organe d'impactage 15 sur une course limitée par le contact de l'épaulement 17 sur la face supérieure libre 3b du bossage 3.

Suivant une forme d'exécution perfectionnée de cette tige intramédullaire, il est possible de prévoir des moyens pour bloquer l'organe d'impactage 15 dans sa position correspondant à l'expansion désirée de l'extrémité proximale de la tige 2. La figure 3 illustre une forme d'exécution de ces moyens qui sont tout simplement constitués par une vis tubulaire 18 vissable dans l'extrémité supérieure 8a, taraudée à cet effet, de la tige 2.

Naturellement, la longueur de la vis tubulaire 18 est déterminée par la position axiale d'impactage de l'organe d'impactage 15 pour qu'elle affleure la face supérieure libre 3b du bossage 3 lorsque l'organe d'impactage occupe la position correspondant l'expansion désirée de l'extrémité proximale 2b de la tige 2.

L'alésage 18a de cette vis tubulaire 18 est taraudé au diamètre et au pas de la vis 7 de fixation de l'embase 6 des pièces complémentaires de cet élément fémoral de prothèse de hanche, de manière à pouvoir permettre la fixation de l'embase 6 du col 5 et de la tête sphérique 4, au moyen de la vis 7, par vissage de cette dernière dans l'alésage taraudé 18a de la vis tubulaire 18.

Suivant une autre caractéristique de l'invention prévue pour faciliter le dévissage éventuel de la tige 2, l'organe d'impactage 15 présente un alésage taraudé 15b permettant la mise en place d'un outil de type connu, tel qu'une masselotte, à l'aide duquel il est possible de déplacer l'organe d'impactage 15 en sens inverse de sa course d'impactage, c'est-à-dire en direction de l'extrémité débouchante du canal 8. Ce déplacement de l'organe d'impactage 15 permet donc de réduire considérablement le couple de dévissage de la tige 2 et, par conséquent, de faciliter grandement son retrait lorsque cela est rendu nécessaire.

Les figures 7 et 8 illustrent une variante d'exécution de cette tige intramédullaire. Selon cette variante, le bossage tronconique prévu pour l'adaptation de l'embase 6 du col 5 et de la tête sphérique 4 de l'élément fémoral à cette tige 2 ne fait pas partie de cette dernière mais lui est fixée par coincement et vis de blocage 7a. Dans cet exemple, l'alésage axial 8 aménagé dans l'extrémité proximale 2b de la tige intramédullaire 2 présente une extrémité débouchante 8b de forme tronconique, dans la paroi de laquelle sont découpées, par six fentes longitudinales débouchantes 19, de répartition angulaire régulière, six languettes 13' disposées à 60° les unes des autres.

Compte tenu de la forme tronconique de l'extrémité débouchante 8b de l'alésage 8, chaque languette 13' présente, sur sa face interne, une rampe 13'a orientée de l'intérieur vers l'extérieur en direction de l'extrémité ouverte du canal 8.

Dans cet exemple, l'organe d'impactage 15' est constitué par un bossage tronconique inférieur d'une pièce de liaison tubulaire 21 qui porte, coaxialement au bossage inférieur 15', le bossage supérieur 3' précité destiné à la fixation de l'embase 6 du col 5 et de la tête sphérique 4.

Les bossages inférieur 15' et supérieur 3' sont séparés l'un de l'autre par une collerette 21a apte à prendre appui sur l'extrémité proximale libre de la tige 2 en fin d'impactage, c'est-à-dire en fin d'engagement, dans l'extrémité supérieure tronconique 8b, de l'alésage 8 de l'organe d'impactage que constitue le bossage inférieur 15' de la pièce de liaison 21.

Naturellement, les dimensions et pentes 13'a des languettes 13 et du bossage tronconique 15' sont déterminées pour que, dans cette position de fin d'impactage, l'extrémité proximale 2b de la tige 2 présente l'expansion radiale désirée, c'est-à-dire correspondant sensiblement à un couple de dévissage, avant ostéogénèse, de l'ordre de 60 N.m.

Pour permettre la fixation de la pièce de liaison 21 et de l'embase (6) de la partie céphalique de cette prothèse à la tige 2, d'une part, l'extrémité inférieure 8c de l'alésage 8 est taraudée au diamètre et au pas de la vis de fixation 7a et, d'autre part, la pièce de liaison 21 présente un alésage axial 21b d'un diamètre au moins égal au diamètre extérieur de la vis 7a pour permettre son vissage dans l'extrémité inférieure 8c de l'alésage 8.

Pour faciliter un dévissage éventuel de la tige intramédullaire 2 et, par conséquent, le retrait de la pièce de liaison 21, au moins l'extrémité supérieure de son alésage 21b présente une partie taraudée 21c permettant l'engagement par vissage d'un outil de retrait, par exemple du type à masselotte, utilisable de la même manière que celui précédemment décrit, pour permettre le dévissage de la tige intramédullaire 2 de l'exemple des figures 1 à 6, par déplacement de l'organe d'impactage 15, en direction de l'extrémité proximale 2b de cette tige 2.

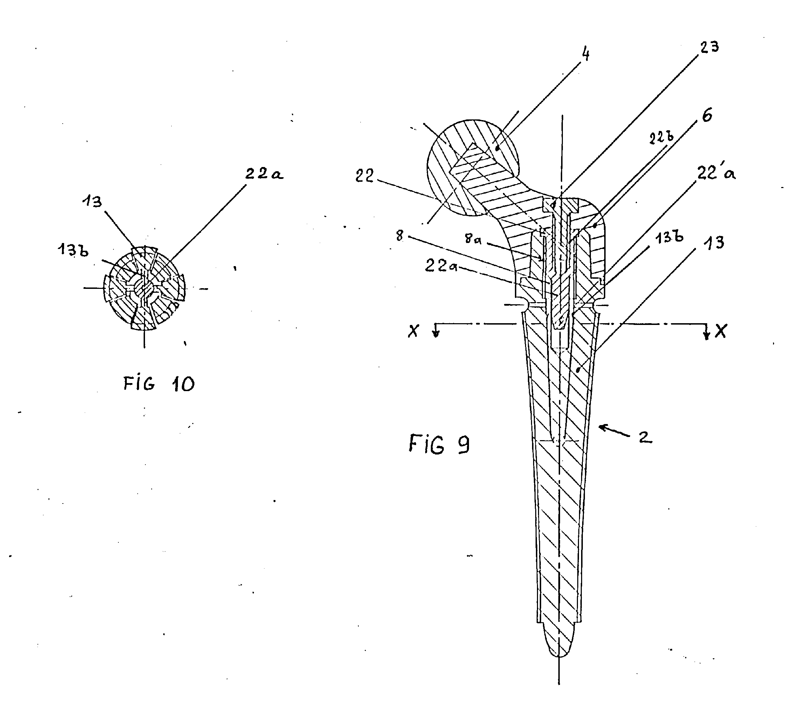

Les figures 9 et 10 illustrent encore une variante d'exécution de cette tige intramédullaire. Dans cet exemple, comme dans celui illustré par les figures 4 à 6, le bossage tronconique 3 prévu pour l'adaptation de l'embase 6 du col 5 et de la tête sphérique 4 de l'élément fémoral à cette tige 2 fait partie intégrante de cette dernière. En outre, comme dans l'exemple précité illustré par les figures 4 à 6, l'extrémité proximale ou débouchante de l'alésage 8 présente un taraudage 8a. De plus, comme dans l'exemple précité, la tige 2 comporte des languettes 13, au nombre de quatre dans cet exemple, mobiles radialement vers l'extérieur en étant solidaires de la tige 2 par leur extrémité distale, c'est-à-dire leur extrémité la plus interne.

Dans cet exemple, l'organe d'impactage est constitué par l'extrémité libre tronconique 22'a d'un prolongement cylindrique lisse 22a d'une vis 22 destinée à être engagée dans l'alésage axial 8 de la tige 2 et vissée dans le taraudage d'extrémité 8a de cet alésage.

Naturellement, pour que le vissage de la vis 22 dans l'alésage 8 de la tige 2 puisse provoquer l'extension radialement vers l'extérieur des languettes 13 dont cette dernière est pourvue, chaque languette 13 présente, sur sa face postérieure, et au voisinage de son extrémité libre, une saillie 13b contre laquelle l'extrémité tronconique 22'a est destinée à être engagée en vue d'en provoquer le déplacement vers l'extérieur et par conséquent l'expansion dans le même sens de chaque languette 13.

Dans cet exemple, l'appui de la tête 22b de la vis 22 contre le fond d'un logement prévu pour la recevoir dans l'extrémité libre du bossage 3 limite son enfoncement dans l'alésage 8 et par conséquent limite l'expansion des languettes 13 a une valeur ne risquant pas de provoquer l'éclatement de l'os.

Comme le montre en outre la figure 10, la vis 22 présente avantageusement un alésage axial taraudé 22c débouchant au centre de sa tête 22b et destiné à recevoir une vis 23 de blocage de l'embase 6 sur le bossage tronconique 3.

Dans l'exemple qui vient d'être décrit, la tige 2 comporte quatre languettes expansives 13, mais on pourrait tout aussi bien en prévoir un nombre différent tel que six par exemple.

Dans les trois exemples qui viennent d'être décrits, la tige intramédullaire 2 porte un filetage extérieur tronconique procurant de meilleurs résultats qu'un filetage cylindrique mais il est évident que l'on pourrait prévoir les mêmes dispositions que celles précédemment décrites en vue de l'expansion de l'extrémité proximale de cette tige si cette dernière portait un filetage cylindrique au lieu d'un filetage tronconique.

## Revendications

**1.-** Tige intramédullaire pour prothèse vissée du type comportant un filetage extérieur cylindrique ou conique, éventuellement recouvert d'une couche d'alumine, **caractérisée en ce qu'**il est prévu, d'une part, un alésage axial (8) débouchant au moins à son extrémité proximale ou externe (2b), dans la paroi de révolution duquel est découpée au moins une languette (13) liée à cette paroi par son extrémité la plus interne et dont la face interne présente une rampe (13a) de pente orientée vers l'extérieur en direction de son extrémité libre et, d'autre part, un organe d'impactage (15) déplaçable axialement depuis l'extrémité extérieure de l'alésage (8) et dont chaque secteur ayant la même position angulaire qu'une languette (13) présente une rampe de pente correspondant à celle de la languette (13) associée, de manière à permettre, par son déplacement axial en direction de l'extrémité distale (2a) de la tige (2), un déplacement radial vers l'extérieur de l'extrémité libre de chaque languette (13), augmentant ainsi le couple de dévissage de cette tige.

**2.-** Tige intramédullaire selon la revendication 1, du type comportant, à son extrémité proximale (2b), un bossage tronconique (3) destiné à recevoir l'embase (6) d'un élément céphalique associé (4,5), le bossage tronconique (3) étant solidaire de l'extrémité proximale (2b) de la tige intramédullaire filetée (2) caractérisée en ce qu'il est prévu, en dessous de ce bossage (3), six fentes longitudinales (9) de répartition angulaire régulière délimitant six secteurs (12,13) de 60° dont trois, non adjacents et destinés à constituer des languettes (13), ont leur extrémité, la plus proche du bossage (3), libérée par une fente en arc de cercle (11), l'organe d'impactage (15) étant constitué par une pièce cylindrique tubulaire (15) présentant, sur sa face externe de révolution, trois secteurs de troncs de cône (15a) disposés à 120° l'un de l'autre et dont chacun est destiné à être applique contre l'une des languettes (13) précitées lors de son engagement dans l'alésage axial (8) de la tige (2), l'alésage (15b) de cet organe d'impactage étant taraudé pour permettre son déplacement axial en sens inverse en cas de dévissage nécessaire de la tige (2).

3.- Tige intramédullaire selon la revendication 2, caractérisée en ce que l'extrémité débouchante ou proximale (8a) de l'alésage (8) de la tige (2) est taraudée et il est prévu une vis tubulaire de blocage (18) apte à être vissée dans cet alésage (8a) jusqu'à son contact avec l'organe d'impactage (15), l'alésage (18a) de cette vis (18) étant taraudé pour permettre la mise en place de la vis de blocage (7) de l'embase (6) de l'élément céphalique (4,5)

4. Tige intramédullaire selon la revendication 1, du type comportant, à son extrémité proximale (2b), un bossage tronconique (3′) destiné à recevoir l'embase (6) d'un élément céphalique associé (4, 5), caractérisée en ce que, d'une part, l'extrémité distale ou non débouchante (8c) de son alésage (8) est taraudée et son extrémité débouchante ou proximale (8b) est tronconique et dans la paroi de cette extrémité, sont découpées, par six fentes longitudinales (19) débouchantes et de répartition angulaire régulière, six languettes (13′) et, d'autre part, l'organe d'impactage (15) est constitué par un bossage inférieur tronconique de pente correspondant à celle de l'extrémité proximale (8b) de l'alésage (8) de la tige (2), solidaire d'une pièce tubulaire de liaison (21) qui porte, coaxialement au bossage (15′), le bossage tronconique supérieur (3′) destiné à recevoir l'embase (6) de l'élément céphalique (4, 5) de la prothèse, l'alésage (21b) de cette pièce (21) étant destiné au passage d'une vis de fixation (7a) de cet élément céphalique (4, 5), apte à être vissée dans l'extrémité distale taraudée (8c) de l'alésage axial (8) de la tige (2), tandis que les deux bossages, inférieur (15′) et supérieur (3′) de cette pièce tubulaire de liaison (21), sont séparés l'un de l'autre par une collerette (21a) destinée à limiter l'enfoncement du bossage inférieur (15′) dans l'extrémité proximale tronconique (8b) de l'alésage (8) de la tige (2) à la valeur correspondant à l'expansion radiale désirée des languettes (13′).

5. Tige intramédullaire selon la revendication 4, caractérisée en ce qu'au moins l'extrémité supérieure (21c) de l'alésage (21b) de la pièce tubulaire de liaison (21) est taraudée.

6. Tige intramédullaire selon la revendication 1, caractérisée en ce que, d'une part, l'organe d'impactage déplaçable axialement est constitué par l'extrémité libre tronconique (22′a) d'un prolongement cylindrique lisse (22a) d'une vis (22) destinée à être vissée dans l'extrémité proximale taraudée (8a) de son alésage axial (8) et, d'autre part, chaque languette (13) porte sur sa face interne et à proximité de son bord libre, une saillie radiale (13b) destinée à être engagée par l'extrémité libre tronconique (22′a) du prolongement lisse (22a) de la vis (22) en fin de vissage de cette dernière.

7. Tige intramédullaire selon la revendication 6, caractérisée en ce que l'extrémité proximale de la vis (22) dont un prolongement lisse (22a) d'extrémité tronconique (22′a) constitue l'organe d'impactage, présente un alésage axial taraudé (22b) destiné à recevoir une vis (23) de blocage de l'embase (6) d'une tête céphalique (4).

## Patentansprüche

1. Intramedullärer Schaft für eine einschraubbare Prothese, versehen mit einem zylindrischen oder konischen Außengewinde, das eventuell mit einer Aluminiumschicht überzogen ist, dadurch gekennzeichnet, daß einerseits eine axiale Bohrung (8) vorgesehen ist, die wenigstens an seinem proximalen oder äußeren Endbereich (2b) mündet, wobei in seine umlaufende Wandung wenigstens eine Zunge (13) geschnitten ist, die mit dieser Wandung über ihr innerstes Ende verbunden ist und deren Innenseite eine Rampe (13a) mit nach außen in Richtung ihres freien Endes gerichteter Schräge aufweist, und andererseits ein Einschlagelement (15) vorgesehen ist, das axial vom äußeren Endbereich der Bohrung (8) her verschiebbar ist und bei dem jeder die gleiche Winkelposition wie eine Zunge (13) aufweisende Sektor eine schräge Rampe aufweist, die der der zugehörenden Zunge (13) entspricht, um durch seine axiale Verschiebung in Richtung des distalen Endbereichs (2a) des Schaftes (2) eine radiale Verschiebung des freien Endbereichs jeder Zunge (13) nach außen zu gestatten, wobei so das Losschraubmoment dieses Schaftes vergrößert wird.

2. Intramedullärer Schaft, mit einem kegelstumpfartigen Vorsprung (3) an seinem proximalen Endbereich (2b), der zur Aufnahme eines Befestigungsansatzes (6) eines damit verbundenen Kopfelements (4,5) bestimmt ist, wobei der kegelstumpfartige Vorsprung (3) zusammenhängend mit dem proximalen Endbereich (2b) des intramedullären, mit einem Gewinde versehenen Schaftes (2) ausgebildet ist, dadurch gekennzeichnet, daß unterhalb dieses Vorsprungs (3) sechs Längsschlitze (9) in regelmäßiger Winkelverteilung vorgesehen sind, die sechs Sektoren (12,13) von 60° begrenzen, von denen drei, die nicht aneinander angrenzen und zur Bildung der Zungen (13) bestimmt sind, an ihrem am nächsten zum Vorsprung (3) hin gelegenen Ende durch einen kreisbogenförmigen Schlitz (11) freigemacht sind, wobei das durch ein Zylinderrohrstück (15) gebildete Einschlagelement (15) an seiner externen umlaufenden Seite drei kegelstumpfartige Sektoren (15a) aufweist, die um 120° zueinander versetzt angeordnet sind und von denen jeder dazu bestimmt ist, gegen eine der oben erwähnten Zungen (13) beim Eingriff in die axiale Bohrung (8) des Schaftes (2) in Anlage zu gelangen, wobei die Bohrung (15b) dieses Einschlagelements mit einem Gewinde versehen ist, um seine axiale Verschiebung in der Gegenrichtung für den Fall des erforderlichen Losschraubens des Schaftes (2) zu ermöglichen.

3.     Intramedullärer Schaft nach Anspruch 2, dadurch gekennzeichnet, daß der Mündungs- oder proximale Endbereich (8a) der Bohrung (8) des Schaftes (2) mit einem Gewinde versehen ist, und es ist eine rohrförmige Sicherungsschraube (18) dazu vorgesehen, in diese Bohrung (8a) bis zum Kontakt mit dem Einschlagelement (15) eingeschraubt zu werden, wobei die Bohrung (18a) dieser Schraube (18) mit einem Gewinde versehen ist, um die Aufnahme der Sicherungsschraube (7) des Befestigungsansatzes (6) des Kopfelements (4,5) zu gestatten.

4.     Intramedullärer Schaft nach Anspruch 1, mit einem kegelstumpfartigen Vorsprung (3′) an seinem proximalen Endbereich (2b), der zur Aufnahme eines Befestigungsansatzes (6) eines damit verbundenen Kopfelements (4,5) bestimmt ist, dadurch gekennzeichnet, daß einerseits der distale oder nicht ausmündende Endbereich (8c) seiner Bohrung (8) mit einem Gewinde versehen und sein Mündungs- oder proximaler Endbereich (8b) kegelstumpfartig ausgebildet ist und in der Wandung dieses Endbereichs durch sechs offen endende Längsschlitze (19) in regelmäßiger Winkelverteilung sechs Zungen (13′) geschnitten sind, und daß andererseits das Einschlagelement (15) durch einen unteren, kegelstumpfartigen, mit einer Schräge entsprechend der des proximalen Endbereichs (8b) der Bohrung (8) des Schaftes (2) versehenen Vorsprung gebildet wird, der mit einem rohrförmigen Verbindungsstück (21) zusammenhängend ausgebildet ist, das koaxial zum Vorsprung (15′) den kegelstumpfartigen oberen Vorsprung (3′) trägt, der zur Aufnahme des Befestigungsansatzes (6) des Kopfelements (4,5) der Prothese bestimmt ist, wobei die Bohrung (21b) dieses Stücks (21) zum Durchlaß einer Befestigungsschraube (7a) dieses Kopfelements (4,5) bestimmt ist, die dazu geeignet ist, in dem distalen, mit einem Gewinde versehenen Endbereich (8c) der axialen Bohrung (8) des Schaftes (2) eingeschraubt zu werden, während die beiden Vorsprünge, der untere (15′) und der obere (3′), dieses rohrförmigen Verbindungsstücks (21) voneinander durch einen Bund (21a) abgeteilt sind, der zur Begrenzung der Eindringtiefe des unteren Vorsprungs (15′) in den proximalen, kegelstumpfartigen Endbereich (8a) der Bohrung (8) des Schaftes (2) auf einen Wert bestimmt ist, der der gewünschten radialen Ausdehnung der Zungen (13′) entspricht.

5.     Intramedullärer Schaft nach Anspruch 4, dadurch gekennzeichnet, daß wenigstens der obere Endbereich (21c) der Bohrung (21b) des rohrförmigen Verbindungsstücks (21) mit einem Gewinde versehen ist.

6.     Intramedullärer Schaft nach Anspruch 1, dadurch gekennzeichnet, daß einerseits das axial verschiebbare Einschlagelement durch den freien kegelstumpfartigen Endbereich (22′a) einer zylindrischen glatten Verlängerung (22a) einer Schraube (22) gebildet wird, die dazu vorgesehen ist, in den proximalen, mit einem Gewinde versehenen Endbereich (8a) seiner axialen Bohrung (8) eingeschraubt zu werden, und daß andererseits jede Zunge (13) an ihrer Innenseite und in der Nähe ihres freien Rands einen radialen Vorsprung (13b) trägt, der dazu vorgesehen ist, mit dem freien kegelstumpfartigen Endbereich (22′a) der glatten Verlängerung (22a) der Schraube (22) am Ende der Verschraubung der letzteren in Anlage zu gelangen.

7.     Intramedullärer Schaft nach Anspruch 6, dadurch gekennzeichnet, daß der proximale Endbereich der Schraube (22), deren glatte Verlängerung (22a) mit dem kegelstumpfartigen Endbereich (22′a) das Einschlagelement bildet, eine axiale, mit einem Gewinde versehene Bohrung (22b) aufweist, die dazu vorgesehen ist, eine Sicherungsschraube (23) des Befestigungsansatzes (6) eines Kopfelements (4) aufzunehmen.

## Claims

1.     An intramedullary rod for a screwed prosthesis of the type having a conical or cylindrical external thread, possibly covered by an aluminium layer, characterised in that there is provided, on the one hand, an axial bore 8 opening at least at its proximal or external end (2b) into the wall of revolution from which is cut at least one tongue (13) connected to this wall by its innermost end and of which the internal face has a ramp (13a) which is outwardly inclined in the direction of its free end and, on the other hand, an impact member (15) which is axially movable from the external end of the bore (8) and of which each sector having the same angular position as a tongue (13) has a ramp of which the inclination corresponds to that of the associated tongue (13), so as to permit, by its axial displacement in the direction of the distal end (2a) of the rod (2), a radially outward displacement of the free end of each tongue (13), thus increasing the unscrewing couple of the rod.

2. An intramedullary rod according to Claim 1, of the type having, at its proximal end (2b), a truncated cone-shaped boss (3) intended to receive the base (6) of an associated cephalic element (4,5), the truncated cone-shaped boss (3) being rigid with the proximal end (2b) of the threaded intramedullary rod (2), characterised in that there is provided, below this boss (3), six longitudinal slots (9), distributed in a regular angular manner, delimiting six sectors (12,13) of 60°, of which three non-adjacent ones intended to constitute tongues (13), have their ends closest to the boss (3) freed by a circular arc-shaped slot (11), the impact member (15) being constituted by a tubular cylindrical part (15) having, on its external face of revolution, three truncated cone-shaped sectors (15a) arranged at 120° from one another and each of which is intended to be applied against one of the aforementioned tongues (13) when it is engaged in the axial bore (8) of the rod (2), the bore (15b) of this impact member being threaded to allow its axial movement in the opposite direction in case of the necessary unscrewing of the rod (2).

3. An intramedullary rod according to Claim 2, characterised in that the end (8a) opening into or proximal to the bore (8) of the rod (2) is threaded and there is provided a tubular locking screw (18) adapted to be screwed into this bore (8a) until it contacts the impact member (15), the bore (18a) of this screw (18) being threaded to allow the insertion of the locking screw (7) of the base (6) of the cephalic element (4,5).

4. An intramedullary rod according to Claim 1, of the type having, at its proximal end (2b), a truncated cone-shaped boss (3') intended to receive the base (6) of an associated cephalic element (4,5), characterised in that, on the one hand, the distal or blind end (8c) of its bore (8) is threaded and its opening or proximal end (8b) is truncated cone-shaped and in the wall of this end are cut, by six open-sided and regularly angularly spaced longitudinal slots (19), six tongues (13') and, on the other hand, the impact member (15) is constituted by a lower truncated cone-shaped boss having an inclination corresponding to that of the proximal end (8b) of the bore (8) of the rod (2), rigid with a tubular connecting part (21) which carries, coaxially with the boss (15'), the upper truncated cone-shaped boss (3') intended to receive the base (6) of the cephalic element (4,5) of the prosthesis, the bore (21b) of this part (21) being intended for the passage of a fixing screw (7a) of this cephalic element (4,5), adapted to be screwed into the threaded distal end (8c) of the axial bore (8) of the rod (2), whilst the two bosses, lower (15') and upper (3') of this tubular connecting part (21), are separated from one another by a collar (21a) intended to limit the penetration of the lower boss (15') into the proximal truncated cone-shaped end (8b) of the bore (8) of the rod (2) to the value corresponding to the desired radial expansion of the tongues (13').

5. An intramedullary rod according to Claim 4, characterised in that at least the upper end (21c) of the bore (21b) of the tubular connecting part (21) is threaded.

6. An intramedullary rod according to Claim 1, characterised in that, on the one hand, the axially movable impact member is constituted by the truncated cone-shaped free end (22'a) of a smooth cylindrical extension (22a) of a screw (22) intended to be screwed into the threaded proximal end (8a) of its axial bore (8) and, on the other hand, each tongue (13) carries on its internal face and close to its free edge, a radial projection (13b) intended to be engaged by the truncated cone-shaped free end (22'a) of the smooth extension (22a) of the screw (22) at the end of the screwing of this latter.

7. An intramedullary rod according to Claim 6, characterised in that the proximal end of the screw (22), of which a smooth extension (22a) having a truncated cone-shaped end (22'a) constitutes the impact member, has a threaded axial bore (22b) intended to receive a locking screw (23) for the base (6) of a cephalic head (4).

## FIG_1

## FIG_2

## FIG_3

FIG.4

FIG.7

FIG.5

FIG.8

FIG.6

FIG 10

FIG 9

EP 0 399 920 B1